(19) Europäisches Patentamt
European Patent Office
Office européen des brevets

(11) **EP 1 491 150 A1**

(12) **DEMANDE DE BREVET EUROPEEN**

(43) Date de publication:
**29.12.2004 Bulletin 2004/53**

(51) Int Cl.7: **A61B 17/17**, A61B 19/00

(21) Numéro de dépôt: **03447172.2**

(22) Date de dépôt: **27.06.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(71) Demandeur: **UNIVERSITE LIBRE DE BRUXELLES
B-1050 Bruxelles (BE)**

(72) Inventeurs:
• **Leloup, Thierry
1160 Bruxelles (BE)**
• **Schuind, Frédéric
3220 Holsbeek (BE)**
• **Warzee, Nadine
1310 La Hulpe (BE)**

(74) Mandataire: **Van Malderen, Joelle et al
Office Van Malderen,
Place Reine Fabiola 6/1
1083 Bruxelles (BE)**

(54) **Procédé d'acquisition d'informations destinées à l'insertion d'une vis de verrouillage dans un orifice d'un objet métallique**

(57)    La présente invention se rapporte à un procédé d'acquisition d'informations destinées à l'insertion d'une vis de verrouillage dans un orifice de verrouillage distal d'un objet métallique, constitué des étapes suivantes :

- Prise de deux images différentes de la partie distale de l'objet métallique à l'aide d'un appareil de scopie,
- Acquisition des paramètres de projection de chaque image en localisant un repère fixé à l'objet métallique et un autre repère fixé audit appareil de scopie,
- Correction des distorsions des images,
- Segmentation de la partie distale de l'objet métallique sur chaque image, fournissant des attributs relatifs à la position dudit objet ainsi qu'à celle des orifices,
- Construction du cône de projection de la partie distale de l'objet pour chaque image,
- Détermination de l'intersection des deux cônes de projection,
- Modélisation de l'objet métallique à partir de l'intersection obtenue dans l'étape précédente,
- Détermination du centre de l'orifice de verrouillage,
- Détermination de l'orientation de l'orifice de verrouillage.

Fig. 2

Printed by Jouve, 75001 PARIS (FR)

**Description**

**Objet de l'invention**

La présente invention se rapporte à un procédé d'acquisition d'informations destinées à l'insertion d'une vis de verrouillage dans un orifice distal d'un objet métallique.

**Etat de la technique**

Les fractures fermées de la diaphyse du fémur et du tibia sont le plus souvent traitées par enclouage centromédullaire. La principale difficulté concernant la technique d'enclouage centromédullaire réside dans la mise en place des vis de verrouillage distales qui peut s'avérer très difficile car les orifices du clou ne sont pas directement visibles par le chirurgien. Les vis proximales sont généralement insérées à l'aide d'un guide mécanique fixé à la poignée d'enclouage. Malheureusement, ce procédé ne peut être utilisé pour la mise en place des vis distales car le clou peut subir une torsion et une flexion importante lors de son insertion pour s'adapter à la forme du canal centromédullaire. L'utilisation de nombreuses images fluoroscopiques est donc inévitable. Plusieurs techniques ont été mises au point pour résoudre ce problème.

La technique classique consiste à positionner l'axe de l'appareil de scopie perpendiculairement aux orifices de verrouillage qui apparaissent ainsi parfaitement circulaires sur les images. Le ciblage s'effectue à l'aide d'un foret ou d'un clou de Steinmann passé à travers un viseur à main libre universel, ou encore à l'aide d'autres systèmes d'assistance mécaniques. Une incision est pratiquée sur la peau, au niveau de l'orifice de verrouillage. La pointe de l'outil est placée contre la paroi corticale externe, au centre de l'orifice et son axe est aligné sur celui de l'amplificateur de brillance. Ceci s'effectue sous contrôle fluoroscopique de face et de profil. Lorsque la position et l'orientation de l'outil sont satisfaisantes, les parois corticales peuvent être percées; la vis est ensuite insérée. Pendant la visée fluoroscopique, l'accès du chirurgien au champ opératoire est souvent limité à cause de la présence de l'appareil de scopie.

Plusieurs autres dispositifs ont été développés pour le verrouillage distal : guides mécaniques de ciblage montés sur la partie proximale du clou, systèmes magnétiques pour localiser le centre de l'orifice de verrouillage... Quelques brevets relatifs à ces techniques ont été déposés (e.g. US-B-5584838, US-A-2002058948, EP-A-1099413, US-B-6200316, US-B-6093192, US-B-5951561, US-B-5411503, US-B-4541424). Cependant aucun ne résout le problème de manière satisfaisante.

La technique de fluoroscopie virtuelle peut également être utilisée. Elle permet de créer l'impression d'une fluoroscopie continue dans une certaine direction sur base d'une seule image. Cette méthode nécessite de suivre la position de la scopie et les outils du chirurgien à l'aide d'un localisateur tridimensionnel et de superposer la projection de ces derniers à l'image acquise initialement en temps réel. Plusieurs vues correspondant à des orientations différentes peuvent être utilisées simultanément. Comme les images fluoroscopiques sont déformées, une correction est nécessaire : celle-ci se base généralement sur une grille de billes ou de croix métalliques. Pour le verrouillage distal, deux vues orthogonales sont acquises, l'une parallèle et l'autre perpendiculaire à l'axe de chaque orifice. Ceci nécessite d'ajuster la position de l'appareil de scopie jusqu'à ce que les trous apparaissent parfaitement circulaires. L'image ainsi obtenue peut servir pour la fluoroscopie virtuelle guidant l'insertion des vis distales.

Certains systèmes utilisent des images tomodensitométriques pour faciliter l'insertion du clou et la réduction de la fracture, mais le verrouillage distal utilise également deux vues qui doivent être prises de face et de profil. Un guide de perçage ajustable passif est fixé à l'extrémité proximale du clou. Il est composé d'une tête radiotransparente et de deux cercles concentriques métalliques dans lesquels vient s'insérer le foret. Sa position est ajustée, comme précédemment, à l'aide de l'amplificateur de brillance pour que l'axe du foret coïncide avec celui de l'orifice de verrouillage.

Une dernière alternative est le système CAOS qui utilise un bras articulé passif, freiné et muni d'un guide de forage pour maintenir l'alignement du moteur de méchage. En effet, le foret peut déraper au contact de l'os cortical, l'axe de l'outil déviant ainsi de celui de l'orifice de verrouillage. Le guidage se base sur une vue fluoroscopique prise face aux trous de verrouillage : le chirurgien doit manipuler le bras articulé pour centrer l'axe du guide de forage avec celui de l'orifice. Le positionnement précis de l'appareil de scopie nécessite cependant la prise de plusieurs clichés d'ajustement.

**Buts de l'invention**

La présente invention vise à fournir un appareil et un procédé d'acquisition d'informations destinées à l'insertion d'une vis de verrouillage distale dans un orifice distal d'un objet métallique et ceci à l'aide d'un système de réalité virtuelle se basant seulement sur deux clichés.

# EP 1 491 150 A1

**Résumé de l'invention**

La présente invention se rapporte à un procédé d'acquisition d'informations destinées à l'insertion d'une vis de verrouillage dans un orifice de verrouillage distal d'un objet métallique, constitué des étapes suivantes :

- Prise de deux images différentes de la partie distale de l'objet métallique à l'aide d'un appareil de scopie,
- Acquisition des paramètres de projection de chaque image en localisant un repère fixé à l'objet métallique et un autre repère fixé à l'appareil de scopie,
- Correction des distorsions des images,
- Segmentation de la partie distale de l'objet métallique sur chaque image, fournissant des attributs relatifs à la position de l'objet ainsi qu'à celle des orifices,
- Construction du cône de projection de la partie distale de l'objet pour chaque image,
- Détermination de l'intersection des deux cônes de projection,
- Modélisation de l'objet métallique à partir de l'intersection obtenue dans l'étape précédente,
- Détermination du centre de l'orifice de verrouillage,
- Détermination de l'orientation de l'orifice de verrouillage.

Selon une forme d'exécution préférée de l'invention cet objet métallique est un clou centromédullaire.

Selon une autre forme d'exécution préférée les images sont des images fluoroscopiques.

Avantageusement les paramètres de projection de chaque image sont acquis à l'aide d'un système optique de localisation 3D.

De préférence une grille de billes métalliques est utilisée afin de corriger les distorsions des images et de calculer automatiquement la position de la source d'irradiation employée.

Selon une forme d'exécution avantageuse le calcul de l'intersection s'effectue à l'aide d'un ensemble de plans parallèles, perpendiculaires à l'axe principal de l'objet.

Typiquement la modélisation de l'objet métallique définit à la fois la surface intérieure et extérieure de ce dernier.

De préférence la détermination du centre de chaque orifice est réalisée à partir de la position de ce centre sur chaque image.

Selon une forme d'exécution spécifique l'orientation de l'orifice de verrouillage est déterminée en envisageant toutes les positions possibles de l'axe de l'orifice et en sélectionnant celle qui fournit le taux de correspondance le plus élevé.

Selon une forme d'exécution alternative la méthode contient également l'étape de la modélisation d'une fente longitudinale de l'objet métallique.

Avantageusement la fente est modélisée par un parallélépipède rectangle.

Selon une forme d'exécution préférée le procédé comprend également une étape de calibrage d'un outil de méchage.

De préférence ce calibrage est réalisé à l'aide d'un repère fixé à l'outil de méchage utilisé.

Un autre aspect de la présente invention est relatif à un dispositif permettant d'acquérir des informations destinées à l'insertion d'une vis de verrouillage dans un orifice distal d'un objet métallique, constitué de

- un appareil de scopie pour prendre deux images de la partie distale dudit objet métallique,
- moyens arrangés pour acquérir des paramètres de projection de chaque image,
- moyens arrangés pour traiter lesdites images et lesdits paramètres en effectuant les étapes suivantes :

  - correction des distorsions desdites images,
  - segmentation de la partie distale dudit objet métallique,
  - construction du cône de projection de la partie distale dudit objet pour chaque image,
  - calcul de l'intersection des deux cônes de projection,
  - modélisation dudit objet à l'aide de ladite intersection,
  - détermination du centre de l'orifice de verrouillage et de son orientation,

- module de navigation pour afficher ledit modèle dudit objet métallique, le module permettant de visualiser le modèle sous un angle compris entre 0° et 360°.

Avantageusement le module de navigation est apte à visualiser le modèle depuis un point de vue appartenant à l'axe d'un orifice dudit objet métallique.

Typiquement les moyens arrangés pour traiter les images et les paramètres sont exécutés dans un produit de programme d'ordinateur.

De préférence le dispositif est également constitué de moyens arrangés pour modéliser une fente longitudinale de l'objet métallique.

**Brève description des figures**

La figure 1 représente un cliché fluoroscopique de la grille de billes métalliques et de l'extrémité distale d'un clou centromédullaire.

La figure 2 représente l'image de la figure 1 après avoir appliqué un seuillage.

La figure 3 représente un cône de projection et différents éléments définis à partir de ce dernier.

La figure 4 illustre la méthode de calcul de l'intersection des deux cônes de projection.

La figure 5 représente un ensemble de contours (polygones) fermés constituant l'intersection des cônes de projection d'un clou centromédullaire.

La figure 6 représente le cercle inscrit à un contour polygonal de la figure 5.

La figure 7 représente le modèle d'un clou constitué d'une surface intérieure et extérieure.

La figure 8 représente l'orientation des axes des orifices à déterminer.

La figure 9 représente, pour chaque orifice, les quatre contours formés par l'intersection des deux cylindres modélisant le clou et du cylindre modélisant l'orifice envisagé.

La figure 10 représente le taux global de correspondance en fonction de l'orientation de l'axe d'un orifice.

La figure 11 représente la modélisation de la fente.

La figure 12 représente quatre vues de guidage de l'outil lors de son alignement avec l'axe de l'orifice.

La figure 13 représente les erreurs d'orientation et de positionnement des axes de deux orifices lors d'essais sur os sec.

La figure 14 représente les trois points considérés pour le calibrage de l'outil de méchage.

**Description détaillée de l'invention**

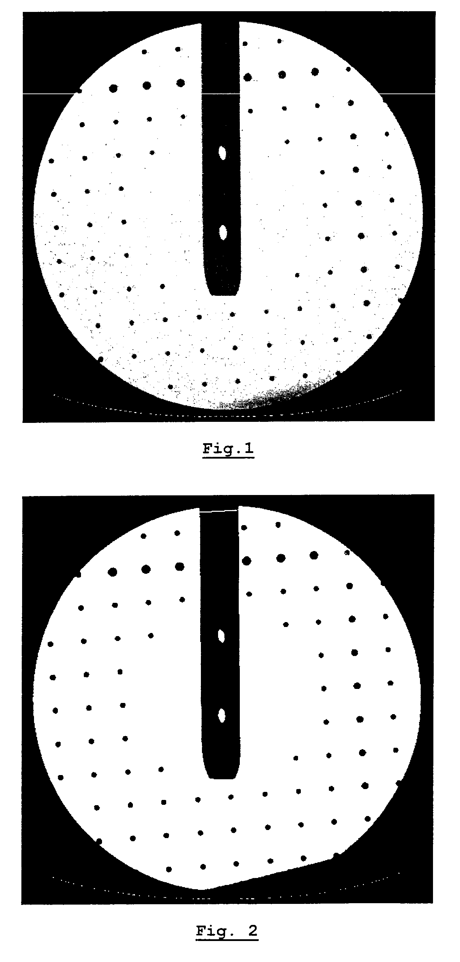

Le système d'acquisition d'informations divulgué dans cette invention nécessite la prise de deux clichés fluoroscopiques sur lesquels les orifices d'insertion des vis distales doivent être visibles. Ces deux vues ne doivent donc pas être effectuées strictement de face et de profil (les orifices peuvent apparaître ovales sur les images). Les paramètres de projection de chaque vue sont acquis à l'aide d'un système optique de localisation 3D. Ils sont constitués de deux repères (corps rigides) : l'un fixé rigidement au clou et l'autre fixé sur une grille de billes métalliques disposée sur l'amplificateur de brillance de l'appareil de scopie. Cette grille est destinée à corriger les distorsions des images fluoroscopiques et peut comporter un second étage permettant de calculer automatiquement la position de la source. Sa partie centrale est dépourvue de billes de manière à ne pas occulter les orifices de verrouillage distaux (Fig. 1). La position de la grille de billes est connue dans le repère qui lui est fixé. Les distorsions des deux images sont corrigées à l'aide d'une méthode classique décrite dans la littérature (modélisation des distorsions par un polynôme bidimensionnel construit grâce à la connaissance des positions des billes métalliques en réalité et sur l'image).

La partie distale du clou centromédullaire est segmentée sur chaque image. A cet effet, l'image est d'abord binarisée à l'aide d'un seuillage (le seuil, dont la valeur peut varier selon l'appareil de scopie utilisé, est choisi empiriquement sur base de quelques images fluoroscopiques et en s'aidant des histogrammes des niveaux de gris de chacune d'entre elles) : les objets métalliques apparaissent donc en noir et le fond de l'image est blanc. Ensuite les pixels situés sur un cercle de rayon légèrement inférieur à celui de l'image sont mis en blanc. Ceci permet de séparer le clou du fond noir de la zone périphérique de l'image (Fig. 2). Tous les objets noirs de l'image sont ensuite étiquetés et trois attributs sont calculés pour chacun d'entre eux : le nombre de contours de l'objet, sa surface et son excentricité. L'objet possédant trois contours (1 contour extérieur et 2 contours intérieurs), ayant une surface plus grande que 2% de la taille de l'image et dont l'excentricité est supérieure à 3,5 est considéré comme le clou. Les coordonnées des pixels constituant ses contours (extérieur et intérieurs) sont enregistrées. D'autres attributs sont également calculés : l'axe principal de l'objet, son centroïde ainsi que ceux des orifices du clou.

L'étape suivante consiste à construire pour chaque vue le cône de projection de la partie distale du clou centromédullaire, exprimé dans le repère fixé au patient. Ceci nécessite de déterminer la transformation linéaire L qui fournit pour tout pixel (x,y), le point tridimensionnel correspondant $L(x,y) = (X,Y,Z)$, exprimé dans le repère fixé à l'amplificateur de brillance, ce qui peut s'écrire sous forme matricielle :

$$L(x,y) = \begin{bmatrix} X \\ Y \\ Z \end{bmatrix} = \begin{bmatrix} M_{11} & M_{12} \\ M_{21} & M_{22} \\ M_{31} & M_{32} \end{bmatrix} \cdot \begin{bmatrix} x \\ y \end{bmatrix} + \begin{bmatrix} T_1 \\ T_2 \\ T_3 \end{bmatrix}$$

où les coefficients de la matrice M et du vecteur T sont à déterminer. A cet effet, les coordonnées de n points $p_i = (x_i, y_i)$ de l'image et des points 3D correspondants $P_i = (X_i, Y_i, Z_i)$ exprimés dans le repère fixé à la scopie doivent être connues. Cette condition peut être satisfaite soit en considérant les coordonnées des billes réelles et les coordonnées des billes présentes sur l'image, soit en considérant une série de points de calibrage (situés dans le plan de la grille de billes) dont la position par rapport au repère de la scopie est enregistrée préalablement et dont la position sur l'image peut être calculée à l'aide des billes présentes sur celle-ci. La connaissance de ces n points correspondants permet de calculer les coefficients recherchés par la méthode des moindres carrés en minimisant la fonction :

$$E = \sum_{i=1}^{n} \left\| P_i - (M \cdot p_i + T) \right\|^2$$

En calculant la norme euclidienne, la fonction E peut s'écrire :

$$E = \sum_{i=1}^{n} (M_{11} \cdot x_i + M_{12} \cdot y_i + T_1 - X_i)^2 + (M_{21} \cdot x_i + M_{22} \cdot y_i + T_2 - Y_i)^2 + (M_{31} \cdot x_i + M_{32} \cdot y_i + T_3 - Z_i)^2$$

En annulant les dérivées partielles de E par rapport à $M_{11}$, $M_{12}$ et $T_1$, on obtient le système de 3 équations à 3 inconnues :

$$\begin{cases} n \cdot T_1 + \sum_{i=1}^{n} x_i \cdot M_{11} + \sum_{i=1}^{n} y_i \cdot M_{12} = \sum_{i=1}^{n} X_i \\[2mm] \sum_{i=1}^{n} x_i \cdot T_1 + \sum_{i=1}^{n} x_i^2 \cdot M_{11} + \sum_{i=1}^{n} x_i \cdot y_i \cdot M_{12} = \sum_{i=1}^{n} x_i \cdot X_i \\[2mm] \sum_{i=1}^{n} y_i \cdot T_1 + \sum_{i=1}^{n} x_i \cdot y_i \cdot M_{11} + \sum_{i=1}^{n} y_i^2 \cdot M_{12} = \sum_{i=1}^{n} y_i \cdot X_i \end{cases}$$

Un raisonnement similaire sur les autres coefficients permet d'obtenir les deux autres systèmes d'équations suivants :

$$\begin{cases} n \cdot T_2 + \sum_{i=1}^{n} x_i \cdot M_{21} + \sum_{i=1}^{n} y_i \cdot M_{22} = \sum_{i=1}^{n} X_i \\[2ex] \sum_{i=1}^{n} x_i \cdot T_2 + \sum_{i=1}^{n} x_i^2 \cdot M_{21} + \sum_{i=1}^{n} x_i \cdot y_i \cdot M_{22} = \sum_{i=1}^{n} x_i \cdot X_i \\[2ex] \sum_{i=1}^{n} y_i \cdot T_2 + \sum_{i=1}^{n} x_i \cdot y_i \cdot M_{21} + \sum_{i=1}^{n} y_i^2 \cdot M_{22} = \sum_{i=1}^{n} y_i \cdot X_i \end{cases}$$

$$\begin{cases} n \cdot T_3 + \sum_{i=1}^{n} x_i \cdot M_{31} + \sum_{i=1}^{n} y_i \cdot M_{32} = \sum_{i=1}^{n} X_i \\[2ex] \sum_{i=1}^{n} x_i \cdot T_3 + \sum_{i=1}^{n} x_i^2 \cdot M_{31} + \sum_{i=1}^{n} x_i \cdot y_i \cdot M_{32} = \sum_{i=1}^{n} x_i \cdot X_i \\[2ex] \sum_{i=1}^{n} y_i \cdot T_3 + \sum_{i=1}^{n} x_i \cdot y_i \cdot M_{31} + \sum_{i=1}^{n} y_i^2 \cdot M_{32} = \sum_{i=1}^{n} y_i \cdot X_i \end{cases}$$

La résolution des ces 3 systèmes de 3 équations à 3 inconnues fournit les coefficients de la matrice M et du vecteur T recherchés.

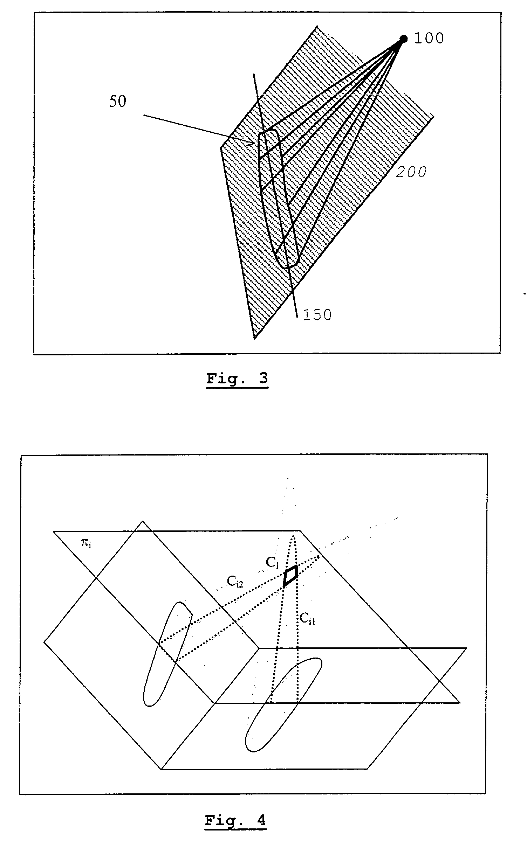

La transformation L permet de convertir le contour bidimensionnel extérieur du clou déterminé sur l'image fluoroscopique en un contour tridimensionnel inclus dans le plan de projection de la vue considérée. En reliant chaque point du contour (50) à la source de rayons X, on obtient le cône de projection du clou centromédullaire pour la vue considérée, exprimé dans le repère lié à la scopie. Lors de la prise du cliché considéré, la position du repère fixé au patient a également été acquise, ce qui permet de calculer la transformation de coordonnées rigide du premier repère vers le second. Le cône de projection considéré peut dès lors être exprimé dans le repère fixé au patient. Pour chaque cône, un plan principal (200) peut être défini (Fig. 3) : il contient l'axe principal (150) de la projection de l'objet et il comprend la source de rayons X (100).

La construction du modèle tridimensionnel du clou consiste dans un premier temps à calculer l'intersection des deux cônes de projection. Ceci est effectué en considérant un ensemble de plans parallèles $\pi_i$, également espacés et perpendiculaires à l'axe principal du clou. L'intersection des plans principaux des cônes de projection fournit une bonne approximation de cet axe. Chaque plan $\pi_i$ coupe le premier cône de projection selon un contour fermé $C_{i1}$. Il en va de même pour le second cône : un contour $C_{i2}$ est défini (Fig. 4). Tous deux sont inclus dans un même plan, ce qui permet de calculer leur intersection :

$$C_{i1} \cap C_{i2} = C_i$$

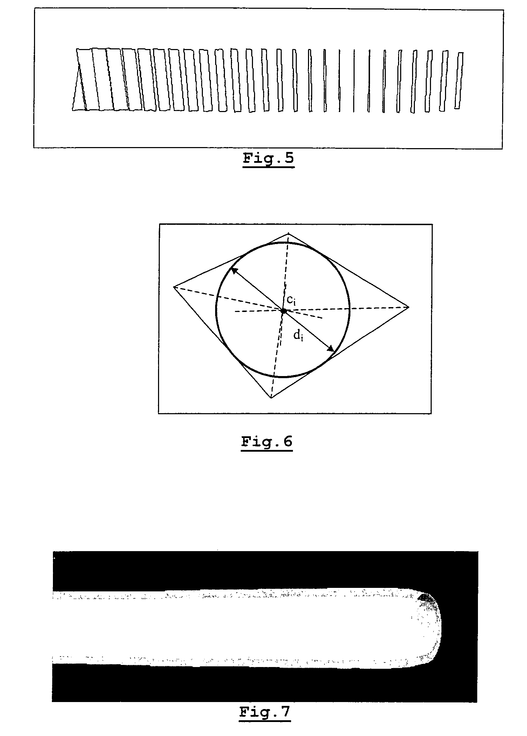

En procédant de la sorte pour chaque plan $\pi_i$, un ensemble $E = \{C_i \,;\, i = 0, ..., n\}$ de contours fermés représentant l'intersection des deux cônes de projection, est obtenu (Fig. 5).

Dans un second temps, le clou est modélisé par un cylindre généralisé : pour chaque contour de l'ensemble E, on calcule le centre $c_i$ et le diamètre $d_i$ du cercle inscrit au polygone considéré (Fig. 6). Ces cercles sont approchés par des polygones réguliers sur lesquels s'appuie un maillage triangulaire modélisant la surface extérieure du clou. Comme l'épaisseur du métal n'est pas négligeable, on modélise également la surface intérieure du clou par un maillage triangulaire s'appuyant sur des cercles de mêmes centres que ceux utilisés précédemment mais dont le rayon a été réduit de l'épaisseur du métal (Fig. 7).

Les centres des orifices de verrouillage du clou centromédullaire sont définis comme les points d'intersection entre l'axe du clou et les axes des orifices, ces derniers étant théoriquement situés dans un même plan. Sur le modèle du clou, les centres des orifices distaux sont donc localisés sur la ligne centrale (ligne reliant les centres des cercles précédemment calculés). La projection du centre de chaque orifice sur l'image fluoroscopique se situe exactement au centre du contour intérieur correspondant du clou segmenté sur l'image. Dès lors, pour localiser les centres des orifices sur la ligne centrale, il suffit de calculer le centre de gravité de chaque contour intérieur du clou sur l'image, de convertir les coordonnées de ces points en coordonnées tridimensionnelles (voir précédemment), de déterminer pour chacun de ces points la droite les rejoignant à la source de rayons X et de calculer les points de la ligne centrale les plus proches de chaque droite. Ainsi, pour chaque vue, on dispose d'une estimation de la position des centres des orifices de verrouillage. Une moyenne des estimations obtenues pour chaque orifice est effectuée, afin de déterminer les coordonnées précises des centres et tenir compte de toute l'information disponible. Cependant, une étape préalable à cette moyenne consiste à distinguer, pour chaque vue, l'orifice le plus distal de l'autre. Ceci est effectué en examinant la proximité de chaque centre avec l'extrémité distale du clou.

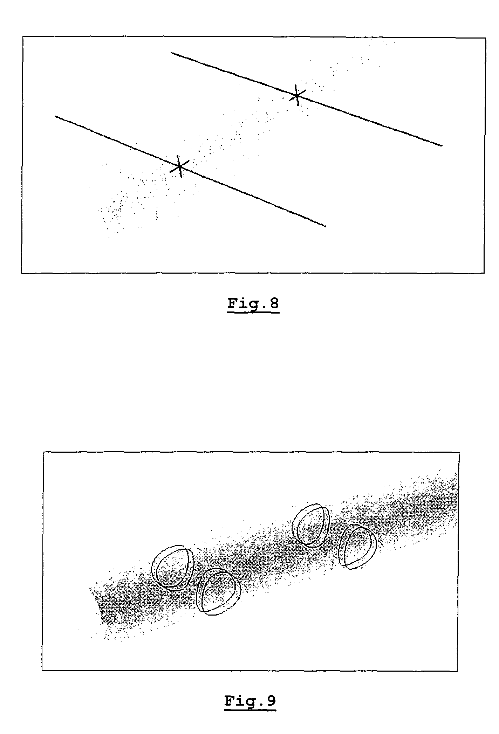

On dispose à présent des cylindres généralisés intérieur et extérieur du clou, de la ligne centrale de celui-ci et des centres des deux orifices de verrouillage. Ces derniers peuvent être modélisés par des cylindres droits dont les axes passent chacun par le centre de l'orifice correspondant et localement perpendiculaires à la ligne centrale. Le diamètre d de ces cylindres peut être mesuré au pied à coulisse. La seule donnée manquante est l'orientation des axes des orifices autour du clou (Fig. 8). Comme le clou peut subir des flexions et des torsions, il faudra envisager séparément la détermination de chacun des deux axes. La méthode consiste à envisager toutes les positions possibles pour l'axe considéré, i.e. toutes les rotations de l'axe autour de la ligne centrale, et à sélectionner celle qui correspond le mieux aux images. Il suffit de prendre en compte un intervalle de rotation de 180° puisque le cylindre est symétrique. Ainsi, pour chaque position considérée de l'axe de l'orifice, un taux de correspondance avec les images fluoroscopiques est calculé. Ce processus nécessite plusieurs étapes:

- Tout d'abord, le cylindre correspondant à l'axe envisagé est construit. Il est constitué de deux cercles (polygones réguliers) de diamètres d (bases du cylindre) et de génératrices parallèles à son axe reliant des points correspondants des deux cercles, également espacés sur ceux-ci. La hauteur h du cylindre est choisie arbitrairement mais doit être supérieure au diamètre du clou.

- Ensuite, les intersections de ce cylindre avec les deux cylindres généralisés modélisant le clou centromédullaire sont déterminées : il s'agit en fait de 4 contours ressemblant à des cercles déformés (Fig. 9). Pour calculer l'intersection d'un cylindre droit modélisant un orifice avec un cylindre généralisé modélisant la surface intérieure ou extérieure le clou, il suffit d'envisager chaque génératrice du cylindre droit et de rechercher ses intersections éventuelles avec les triangles du cylindre généralisé considérés séparément. Le problème se réduit donc au calcul de l'intersection entre une droite et un triangle.

- L'étape suivante consiste à projeter ces contours sur le plan de projection de chaque vue. A cet effet, il suffit de projeter les points des contours un par un en utilisant les transformations de coordonnées inverses de celles décrites plus haut. L'intersection de ces 4 contours bidimensionnels forme un nouveau contour correspondant à l'orifice qui serait visible sur l'image si l'orientation de son axe était celle considérée.

- Enfin, ce contour (appelons-le CE) est comparé à celui de l'orifice correspondant, effectivement présent sur l'image (que l'on notera $C_I$). Leur intersection est déterminée et constitue un contour $C_{EI}$. Si l'on note par A(C) l'aire de la surface intérieure du contour fermé C, on peut calculer un taux de correspondance entre les contours CE et CI pour l'image i considérée :

$$t_i = \frac{2 \cdot A(C_{EI})}{A(C_E) + A(C_I)}$$

Un taux global de correspondance est obtenu en effectuant la moyenne des taux de correspondance $t_i$ correspondant aux 2 vues fluoroscopiques :

$$t_G = \frac{1}{2}(t_1 + t_2)$$

Comme le taux de correspondance d'une image est toujours compris entre 0 et 1, il en va de même pour le taux global de correspondance.

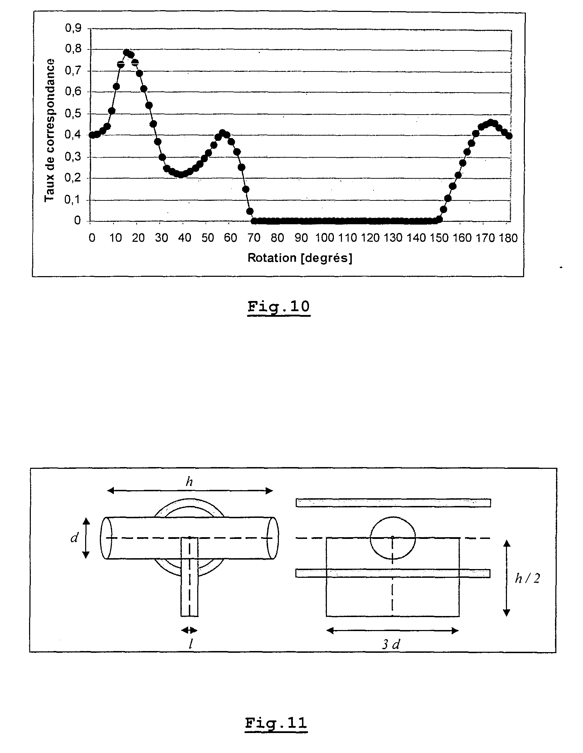

En répétant ce processus pour chaque orientation considérée de l'axe et en enregistrant le taux obtenu à chaque itération, on peut représenter le graphique du taux global de correspondance de l'orifice calculé avec l'orifice réel en fonction de l'orientation du premier (Fig. 10). Il suffit dès lors de déterminer le taux global de correspondance le plus élevé pour connaître l'orientation réelle de l'axe de l'orifice de verrouillage considéré. La même méthode est appliquée pour déterminer l'orientation de l'axe du second orifice. Les 8 contours d'intersection des cylindres droits modélisant les orifices de verrouillage avec les cylindres intérieur et extérieur du clou correspondant au taux maximum global de correspondance sont enregistrés pour pouvoir être affichés par la suite.

La méthode envisagée permet également de modéliser une fente longitudinale du clou. Il est relativement aisé d'introduire les données de la fente longitudinale dans la modélisation du clou centromédullaire. En effet, sur une coupe transversale du clou, la direction de la fente est perpendiculaire à l'axe des orifices de verrouillage. Comme le clou peut avoir été déformé lors de son insertion, elle n'est modélisée que sur une courte distance, séparément pour chaque orifice. Les modifications à apporter au processus de recherche de l'orientation de l'axe d'un orifice décrit dans le paragraphe précédent sont décrites ci-dessous.

La fente correspondant à l'orifice considéré est modélisée par un parallélépipède rectangle qui s'arrête à la moitié du clou et qui ne le traverse pas de part et d'autre comme les cylindres droits modélisant les orifices. Un des axes de ce parallélépipède correspond à la direction locale du clou déterminé par les points de sa ligne centrale dans le voisinage du centre de l'orifice considéré. Son second axe correspond à celui de l'orifice. Ceci détermine la direction du troisième axe qui est en fait la direction de la fente elle-même. Une des faces de ce parallélépipède est centrée sur le centre de l'orifice, la face opposée, parallèle à la première, se situe à une distance supérieure au plus grand rayon du cylindre généralisé modélisant la surface extérieure du clou, de manière à s'assurer que le parallélépipède traverse les deux cylindres généralisés. Sa largeur $l$ dépend de la largeur de la fente. On ne doit modéliser la fente que dans le voisinage de l'orifice considéré, par exemple sur une longueur de 3 fois le diamètre d. Ces mesures déterminent complètement la taille et la position du parallélépipède (Fig. 11).

Comme pour les cylindres droits modélisant les orifices, son intersection avec les cylindres généralisés modélisant le clou est ensuite déterminée, ce qui fournit deux contours supplémentaires par orifice. Les 6 contours sont ensuite groupés par paires : les deux contours correspondant à la fente, les deux contours de l'orifice situés d'un côté du clou et les deux derniers situés de l'autre côté. Ces contours sont ensuite projetés sur le plan d'une image et l'on détermine l'intersection des contours appartenant à la même paire. Ceci fournit 3 contours $C_{O1}$, $C_{O2}$ et $C_F$, ce dernier correspondant à l'intersection de la paire de contours provenant de la fente du clou. Comme il est impossible qu'un rayon X traverse simultanément l'orifice de part et d'autre et la fente du clou, il est inutile de calculer l'intersection de ces 3 contours. Par contre, les rayons X ne rencontreront pas de métal dans trois situations :

- s'ils traversent l'orifice de part et d'autre,
- s'ils traversent la fente et une extrémité de l'orifice,
- s'ils traversent la fente et l'autre extrémité de l'orifice.

Ceci amène à considérer les intersections des surfaces déterminées par les contours $C_{O1}$, $C_{O2}$ et $C_F$, pris deux à deux. L'ensemble E de ces intersections représente sur l'image considérée les zones intérieures au contour du clou où les rayons X n'ont pas rencontré de métal pour l'orientation considérée de l'axe de l'orifice. Il reste donc à comparer E à l'orifice effectivement détecté sur l'image, de contour CI, en calculant l'intersection de E et de $C_I$. En adoptant les mêmes notations que précédemment, le taux de correspondance de l'orientation envisagée et de l'orientation réelle de l'ensemble 'orifice+fente' est donné par :

$$t_i = \frac{2 \cdot A(E)}{A(E) + A(C_I)}$$

où

$$E = (C_{O1} \cap C_{O2}) \cup (C_{O1} \cap C_F) \cup (C_{O2} \cap C_F)$$

et

$$A(E) = A(C_{O1} \cap C_{O2}) + A(C_{O1} \cap C_F) + A(C_{O2} \cap C_F)$$

La moyenne des $t_i$ obtenus pour les deux images donne la valeur du taux de correspondance globale de l'orientation considérée avec la situation réelle.

Signalons toutefois que dans ce cas, l'examen de l'orientation de l'orifice considéré doit être effectué sur un intervalle de 360° car l'ensemble de la modélisation 'orifice + fente' n'est plus symétrique par rapport à l'axe du clou.

Un autre objet de l'invention vise à fournir un logiciel de navigation permettant d'afficher le modèle tridimensionnel du clou centromédullaire. Le modèle peut être visualisé sous n'importe quel angle, mais les situations les plus intéressantes sont celles où il est examiné depuis un point de vue appartenant à l'un des axes des orifices ('vue de guidage'). Dans un tel cas, les 4 contours de l'orifice concerné apparaissent selon des cercles concentriques. Une fonction spéciale permet de visualiser automatiquement le clou de cette manière pour chaque orifice et depuis chacun de ses côtés.

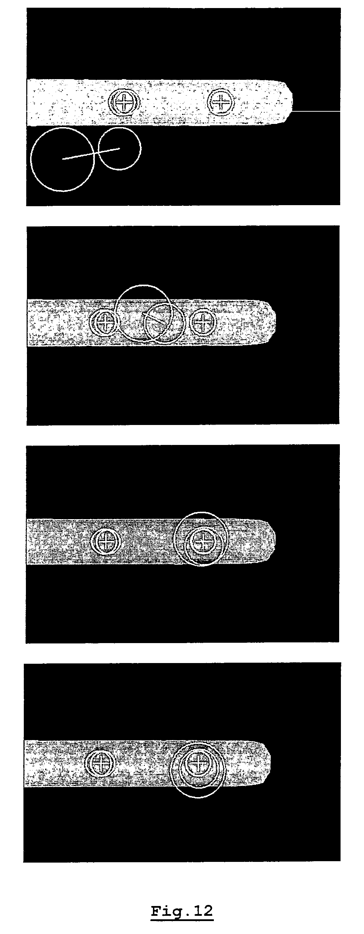

Un repère est fixé à l'outil de méchage et un processus de calibrage permet de déterminer exactement la position de l'extrémité de la mèche et son orientation par rapport au repère de l'outil. Lors de la procédure de guidage du geste chirurgical, un segment de droite représentant la mèche de l'outil est affiché en temps réel. En outre, deux cercles ayant pour centres les extrémités de ce segment et étant situés dans des plans perpendiculaires à ce dernier sont également affichés de façon à faciliter l'alignement de l'outil sur l'axe de l'orifice lorsque le chirurgien dispose d'une vue de guidage. En effet, dans un tel cas, il doit faire en sorte que le segment modélisant l'outil devienne un point centré sur l'orifice considéré, ce qui implique qu'il devient presque invisible. La présence des deux cercles permet une autre approche : pour positionner son outil, le chirurgien peut faire abstraction du segment modélisant la mèche de l'outil et considérer uniquement les cercles, le but à atteindre étant de les rendre tous deux concentriques et centrés sur l'orifice. En pratique, on utilise les deux approches : dans un premier temps, le chirurgien positionne son outil en se repérant au segment et essaie de le réduire à un point centré sur l'orifice; dans un second temps, lorsque le segment devient presque invisible, l'utilisation des deux cercles s'avère beaucoup plus facile (Fig. 12).

Lors du guidage, les positions du repère fixé au clou et de celui attaché à l'outil sont numérisées grâce au localisateur tridimensionnel. La transformation de coordonnées du second repère vers le premier est ensuite déterminée. Les extrémités du segment modélisant l'outil sont connues dans le repère de ce dernier. En appliquant la transformation calculée précédemment à ces deux points, on obtient les coordonnées des extrémités du segment dans le repère du clou, ce qui permet d'afficher l'outil sur l'image tridimensionnelle. Ce processus est effectué en boucle. Le clou reste donc immobile, seul l'outil se déplace à l'écran.

### Exemple 1 : Expérimentations sur un clou seul

Plusieurs expérimentations du système de navigation ont été effectuées. Le dispositif expérimental adopté pour chaque essai est décrit ci-dessous.

Un clou centromédullaire est fixé à la table d'opération par sa partie proximale. La fente du clou est orientée vers le bas, comme lors d'une opération réelle (le patient étant sur le dos, la concavité du clou doit être orientée vers le bas), les axes des orifices étant approximativement horizontaux. Le support de cassettes radiographiques est fixé sur l'amplificateur de brillance et le plateau de polycarbonate contenant les billes de la grille y est inséré. Deux pinces permettent en outre d'éviter d'éventuels déplacements de la grille dus à un faible jeu existant entre celle-ci et le support. L'appareil de fluoroscopie est installé pour pouvoir acquérir des vues où les orifices de verrouillages distaux du clou apparaissent dans la zone de la grille dépourvue de billes. Un repère (étoile suivie par le localisateur 3D) est fixé rigidement à la partie proximale du clou centromédullaire. Au lieu de fixer un autre repère à la grille, 4 points de calibrages ont été définis et sont acquis à l'aide d'un pointeur lorsque la position de la grille doit être connue. Comme une grille simple a été utilisée, la position de la source de rayons X a également été acquise à l'aide du pointeur (numérisation de deux points situés de part et d'autre de la source).

Les 6 points destinés à calculer les paramètres de projection de la scopie sont acquis et le cliché correspondant est effectué puis enregistré. Le second cliché est obtenu de la même façon. Ces images sont ensuite corrigées et les contours du clou sont déterminés sur chaque vue, permettant la construction tridimensionnelle du modèle de ce dernier. L'appareil de fluoroscopie, devenu inutile, est mis à l'écart.

Dans le but d'éviter une déstérilisation inutile d'un moteur de méchage, on a modélisé l'outil par une longue vis de verrouillage. Un repère est également fixé rigidement à celle-ci à l'aide d'une articulation de fixateur externe. Cette vis possède un diamètre de 6,28 mm alors que la mèche utilisée normalement avec la foreuse mesure 5 mm de diamètre. Ceci permet de caler la vis munie de son repère dans un orifice de verrouillage du clou étant donné le faible jeu (0,22 mm) et d'acquérir ainsi la position et l'orientation réelle de l'orifice. Le calibrage de l'outil s'effectue en numérisant la tête de la vis et son autre extrémité à l'aide du pointeur 3D.

Ces différentes données sont finalement chargées dans le logiciel de navigation, la vue tridimensionnelle souhaitée est sélectionnée et le guidage peut commencer. Si l'opérateur le souhaite, les images fluoroscopiques corrigées peuvent être affichées à côté de la vue tridimensionnelle. L'axe de l'outil est projeté sur celles-ci et modifié en temps réel en fonction de sa position.

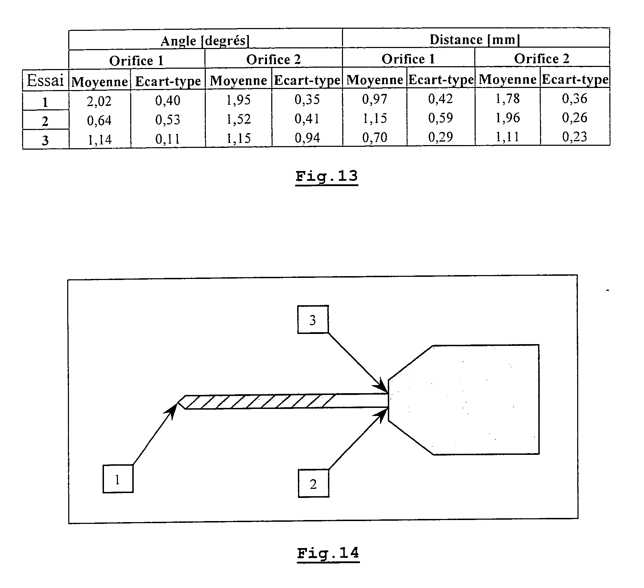

Trois essais différents ont été effectués pour lesquels la position de l'axe de l'outil a été enregistrée lorsqu'il était inséré dans les orifices de verrouillage. Comme le jeu entre l'outil et l'orifice est très faible, cet axe fournit une bonne mesure des positions et de l'orientation réelles des orifices. Afin de quantifier l'erreur de modélisation des orifices, on a comparé les axes réels avec ceux calculés au cours de notre modélisation. Pour chaque orifice, l'angle a été calculé formé par l'axe réel et l'axe calculé ainsi que la distance séparant l'axe réel du centre calculé de l'orifice. Plusieurs positions de l'outil ont été enregistrées pour un même orifice et les moyennes et écarts-types des erreurs ont été calculées (Fig. 13). On constate que l'erreur d'orientation des axes des orifices est de l'ordre de 2° et que l'erreur de positionnement est de l'ordre de 2 mm.

Le temps de calcul nécessaire à la correction d'une image fluoroscopique n'excède pas 30 secondes et celui requis pour la segmentation du clou sur une image corrigée est du même ordre de grandeur. La construction du modèle tridimensionnel du clou et de ses orifices s'effectue en 2 minutes environ. Ces délais sont tout à fait acceptables pour l'opération considérée.

## Exemple 2 : Expérimentations sur un os sec

Un fémur en plastique a été scié en deux transversalement et le fragment distal a été alésé à l'aide d'une foreuse. L'extrémité distale du clou a été insérée dans ce fragment sur une longueur d'environ 10 cm.

Un moteur de méchage universel équipé d'une mèche de 5 mm de diamètre a été utilisé pour percer le trou de la vis de verrouillage. Un repère a été fixé sur la poignée de l'outil à l'aide de colliers de serrage en nylon. Une pièce de caoutchouc insérée entre les deux éléments évite un glissement de ceux-ci. Le calibrage de l'outil s'effectue en repérant trois points à l'aide du pointeur (Fig. 14): l'extrémité de la mèche (1) et deux points diamétralement opposés situés sur le mandrin de l'outil (2-3). L'axe de la mèche est déterminé par le segment reliant le premier point et le milieu des deux derniers. Mis à part cela, le dispositif expérimental est identique à celui des essais sur un clou seul.

On a constaté que pour 94% des essais, le système de navigation a permis de verrouiller le clou. Dans 70% des cas, la mèche a directement traversé les deux trous de l'orifice. Un essai (sur 17) a échoué car l'opérateur a déplacé le corps rigide par rapport à l'outil en appuyant sur celui-ci. Après un recalibrage de l'outil, la mèche a pu traverser le clou sans problème.

Les images utilisées par le système divulgué dans cette invention ne doivent pas être prises de face et de profil (les orifices de verrouillage ne doivent pas apparaître parfaitement circulaires sur l'une des deux images). La seule contrainte réside dans la visibilité des orifices distaux sur chacune des deux images. L'ajustement de la position de l'appareil de scopie s'en trouve simplifié, ce qui réduit l'irradiation du patient et de l'équipe chirurgicale. De plus, le chirurgien dispose d'une vue en trois dimensions, ce qui permet de visualiser simultanément plusieurs vues du clou et de l'outil sous n'importe quels angles, une extrapolation de la trajectoire de la mèche peut être affichée à l'écran et l'erreur de positionnement initial peut être calculée et fournie au chirurgien avant insertion de la vis.

Le procédé de l'invention ne se limite pas à l'exemple décrit précédemment. Le même procédé peut s'appliquer par exemple dans le secteur de la mécanique, où dans certaines applications il faut insérer avec haute précision une vis dans des endroits difficiles à atteindre.

## Revendications

**1.** Procédé d'acquisition d'informations destinées à l'insertion d'une vis de verrouillage dans un orifice de verrouillage distal d'un objet métallique, constitué des étapes suivantes :

- Prise de deux images différentes de la partie distale dudit objet métallique à l'aide d'un appareil de scopie,
- Acquisition des paramètres de projection de chaque image en localisant un repère fixé audit objet métallique et un autre repère fixé audit appareil de scopie,
- Correction des distorsions des images,
- Segmentation de la partie distale dudit objet métallique sur chaque image, fournissant des attributs relatifs à la position dudit objet ainsi qu'à celle des orifices,
- Construction du cône de projection de la partie distale de l'objet pour chaque image,
- Détermination de l'intersection des deux cônes de projection,
- Modélisation dudit objet métallique à partir de ladite intersection obtenue dans l'étape précédente,
- Détermination du centre de l'orifice de verrouillage,
- Détermination de l'orientation de l'orifice de verrouillage.

**2.** Procédé selon la revendication 1, **caractérisé en ce que** ledit objet métallique est un clou centromédullaire.

**3.** Procédé selon la revendication 1 ou 2, **caractérisé en ce que** lesdites images sont des images fluoroscopiques.

**4.** Procédé selon l'une quelconque des revendications 1 à 3, **caractérisé en ce que** lesdits paramètres de projection de chaque image sont acquis à l'aide d'un système optique de localisation 3D.

**5.** Procédé selon l'une quelconque des revendications 1 à 4, **caractérisé en ce que** une grille de billes métalliques est utilisée afin de corriger les distorsions des images et de calculer automatiquement la position de la source d'irradiation employée.

**6.** Procédé selon l'une quelconque des revendications 1 à 5, **caractérisé en ce que** le calcul de ladite intersection s'effectue à l'aide d'un ensemble de plans parallèles, perpendiculaires à l'axe principal dudit objet.

**7.** Procédé selon l'une quelconque des revendications 1 à 6, **caractérisé en ce que** la modélisation dudit objet métallique définit à la fois la surface intérieure et extérieure de l'objet.

**8.** Procédé selon l'une quelconque des revendications 1 à 7, **caractérisé en ce que** la détermination du centre de chaque orifice est réalisée à partir d'une estimation de la position de ce centre sur chaque image.

**9.** Procédé selon l'une quelconque des revendications 1 à 8, **caractérisé en ce que** l'orientation de l'orifice de verrouillage est déterminée en envisageant toutes les positions possibles de l'axe de l'orifice et en sélectionnant celle qui fournit le taux de correspondance le plus élevé.

**10.** Procédé selon l'une quelconque des revendications 1 à 9, **caractérisé en ce que** la méthode contient également une étape de la modélisation d'une fente longitudinale dudit objet métallique.

**11.** Procédé selon la revendication 10, **caractérisé en ce que** ladite fente est modélisée par un parallélépipède rectangle.

**12.** Procédé selon l'une quelconque des revendications 1 à 11, **caractérisé en ce que** le procédé comprend une étape de calibrage d'un outil de méchage.

**13.** Procédé selon la revendication 12, **caractérisé en ce que** le calibrage est réalisé à l'aide d'un repère fixé à l'outil de méchage utilisé.

**14.** Dispositif permettant d'acquérir des informations destinées à l'insertion d'une vis de verrouillage dans un orifice distal d'un objet métallique, constitué de

- un appareil de scopie pour prendre deux images de la partie distale dudit objet métallique,
- moyens arrangés pour acquérir des paramètres de projection de chaque image,
- moyens arrangés pour traiter lesdites images et lesdits paramètres en effectuant les étapes suivantes :

  • correction des distorsions desdites images,
  • segmentation de la partie distale dudit objet métallique,
  • construction du cône de projection de la partie distale dudit objet pour chaque image,
  • calcul de l'intersection des deux cônes de projection,
  • modélisation dudit objet à l'aide de ladite intersection,
  • détermination du centre de l'orifice de verrouillage et de son orientation,

- module de navigation pour afficher ledit modèle dudit objet métallique, le module permettant de visualiser le modèle sous un angle compris entre 0° et 360°.

**15.** Dispositif selon la revendication 14, **caractérisé en ce que** ledit module de navigation est apte à visualiser le modèle depuis un point de vue appartenant à l'axe d'un orifice dudit objet métallique.

**16.** Dispositif selon la revendication 14, **caractérisé en ce que** lesdits moyens arrangés pour traiter lesdites images et lesdits paramètres sont exécutés dans un produit de programme d'ordinateur.

**17.** Dispositif selon la revendication 14, **caractérisé en ce qu'**il est également constitué de moyens arrangés pour modéliser une fente longitudinale dudit objet métallique.

Fig.1

Fig. 2

Fig. 3

Fig. 4

Fig.5

Fig.6

Fig.7

**Fig.8**

**Fig.9**

Fig.10

Fig.11

Fig.12

| Essai | Angle [degrés] | | | | Distance [mm] | | | |
|---|---|---|---|---|---|---|---|---|
| | Orifice 1 | | Orifice 2 | | Orifice 1 | | Orifice 2 | |
| | Moyenne | Ecart-type | Moyenne | Ecart-type | Moyenne | Ecart-type | Moyenne | Ecart-type |
| 1 | 2,02 | 0,40 | 1,95 | 0,35 | 0,97 | 0,42 | 1,78 | 0,36 |
| 2 | 0,64 | 0,53 | 1,52 | 0,41 | 1,15 | 0,59 | 1,96 | 0,26 |
| 3 | 1,14 | 0,11 | 1,15 | 0,94 | 0,70 | 0,29 | 1,11 | 0,23 |

Fig.13

Fig.14

**Office européen des brevets**

## RAPPORT DE RECHERCHE EUROPEENNE

Numéro de la demande

EP 03 44 7172

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 6 285 902 B1 (LEA JON T ET AL) 4 septembre 2001 (2001-09-04) | 14-17 | A61B17/17 A61B19/00 |
| Y | * colonne 9, ligne 18-22 - colonne 10, ligne 52-55; revendications 1,2,4,6,21,23,27; figures 4,5,18 * * colonne 26, ligne 51-53 * * colonne 27, ligne 6-9 - colonne 28, ligne 3-9 * | 1-13 | |
| X | DE 100 37 491 A (STRYKER LEIBINGER GMBH & CO KG) 14 février 2002 (2002-02-14) | 14-17 | |
| Y | * alinéas [0022],[0034],[0040],[0041],[0052]; figures 1-5,11 * | 1-13 | |
| X | HOFSTETTER R ET AL: "COMPUTER-ASSISTED FLUOROSCOPY-BASED REDUCTION OF FEMORAL FRACTURES AND ANTETORSION CORRECTION" COMPUTER AIDED SURGERY, XX, XX, vol. 5, 2000, pages 311-325, XP001001432 | 14-17 | |
| A | * page 312 - page 322; figures 1,2 * | 1-13 | DOMAINES TECHNIQUES RECHERCHES (Int.Cl.7) |
| X | US 4 899 318 A (SCHLUMBERGER ETIENNE ET AL) 6 février 1990 (1990-02-06) | 14-17 | A61B G06T |
| A | * alinéa [0024]; revendications 1,3; figure 1 * | 1-13 | |
| A | US 2003/088179 A1 (LIN FAITH ET AL) 8 mai 2003 (2003-05-08) * alinéas [0039],[0037],[0055]; figures 1,3,7 * | 1-17 | |

Le présent rapport a été établi pour toutes les revendications

| Lieu de la recherche | Date d'achèvement de la recherche | Examinateur |
|---|---|---|
| MUNICH | 20 novembre 2003 | Assion, J-C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.........................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**          EP 03 44 7172

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-11-2003

| Document brevet cité au rapport de recherche | | Date de publication | Membre(s) de la famille de brevet(s) | | Date de publication |
|---|---|---|---|---|---|
| US 6285902 | B1 | 04-09-2001 | AU | 3857900 A | 29-08-2000 |
| | | | EP | 1158891 A2 | 05-12-2001 |
| | | | WO | 0047103 A2 | 17-08-2000 |
| | | | US | 2001036245 A1 | 01-11-2001 |
| DE 10037491 | A | 14-02-2002 | DE | 10037491 A1 | 14-02-2002 |
| | | | WO | 0209611 A2 | 07-02-2002 |
| | | | EP | 1259185 A2 | 27-11-2002 |
| US 4899318 | A | 06-02-1990 | FR | 2624634 A1 | 16-06-1989 |
| | | | AT | 115310 T | 15-12-1994 |
| | | | CA | 1300368 C | 12-05-1992 |
| | | | DE | 3852386 D1 | 19-01-1995 |
| | | | EP | 0323770 A1 | 12-07-1989 |
| | | | IN | 171941 A1 | 13-02-1993 |
| | | | JP | 2042585 A | 13-02-1990 |
| US 2003088179 | A1 | 08-05-2003 | US | 6490475 B1 | 03-12-2002 |
| | | | AU | 5738401 A | 26-11-2001 |
| | | | CA | 2407616 A1 | 22-11-2001 |
| | | | EP | 1278458 A2 | 29-01-2003 |
| | | | WO | 0187136 A2 | 22-11-2001 |

EPO FORM P0460

Pour tout renseignement concernant cette annexe : voir Journal Officiel de l'Office européen des brevets, No.12/82